# EUROPEAN PATENT APPLICATION

(11) **EP 2 371 415 A1**
(43) Date of publication of application: **05.10.2011**
(21) Application number: 09834453.4
(22) Date of filing: 24.12.2009
(51) Int. Cl.: A61M 25/01, A61M 25/00

(54) **CATHETER**

(30) Priority: 26.12.2008 JP 2008332204; 27.04.2009 JP 2009108473
(71) Applicant: Sumitomo Bakelite Company Limited, Shinagawa-ku Tokyo 140-0002 (JP)
(72) Inventor: TANAKA, Hayao, Akita-shi Akita 011-8510 (JP); KANEMASA, Kenichi, Tokyo 140-0002 (JP); SAKATA, Yoichi, Tokyo 140-0002 (JP)
(74) Representative: Vossius, Corinna
(86) International application number: PCT/JP2009/007158
(87) International publication number: WO 2010/073646

(57) **Abstract**

A catheter (10) has a main lumen (20), and sub-lumens (30) each of which having a diameter smaller than that of the main lumen (20) . The plurality of sub-lumens (30) are arranged along a circumference of the main lumen (20) in a discrete manner, and each of the sub-lumens (30) has an operating wire (40), fixed to the distal end section (15) of the catheter (10), laid therethrough in a freely slidable manner. By pulling the operating wire(s) (40) respectively laid through the sub-lumens (30), the distal end section (15) of the catheter (10) may be bent towards the pulled operating wire(s) (40) laid therethrough.

## Description

### TECHNICAL FIELD

The present invention relates to a catheter.

### BACKGROUND ART

Recently, there has been proposed a catheter, a direction of insertion of which into a body cavity is controllable by allowing a distal end section thereof to bend. With respect to this sort of technique, Patent Document 1 below describes one embodiment of bending of the distal end of the catheter, configured to operate a push/pull wire fixed to the distal end, at a proximal end section.
The push/pull wire is laid through a wire lumen, which has a diameter smaller than that of a main lumen having a guide wire laid therethrough. According to the description, the distal end of the catheter may be bent towards the wire lumen by pulling the push/pull wire, and towards the opposite side by pushing it.

### RELATED DOCUMENTS

### PATENT DOCUMENT

[Patent Document 1] Published Japanese Translation of PCT International Publication for Patent Application No. 2007-507305

### DISCLOSURE OF THE INVENTION

The invention described in the Patent Document has been suffering from two problems below.
A first problem relates to that the distal end of the catheter does not always bend towards the direction opposite to the wire lumen, when the push/pull wire is pushed into the catheter. More specifically, the distal end of the catheter is expected to bend towards the wire lumen, when the push/pull wire was pulled, whereas the distal end will only remain straightened without allowing itself to bend towards the opposite side of the wire lumen, when the push/pull wire is pushed into the catheter. Assuming now that even if the distal end should bend towards the opposite direction of the wire lumen as a result of pressing of the push/pull wire, the resultant degree of flexion is very small, and is even asymmetric when compared between pushing and pulling of the wire. It is therefore difficult for the above-described invention to attain a sufficient level of control in the direction of advancement of the catheter.
A second problem arises from that the push/pull wire has a rigidity enough to allow the distal end of the catheter to bend when the push/pull wire is pushed. Accordingly, if an excessive force of pushing should occur at the distal end as a result of pushing by the operator, the push/pull wire is anticipated to break through the distal end of the catheter into the body cavity.

The present invention was conceived after considering the above-described problems, so as to provide a catheter capable of being controlled in the direction of advancement in a desirable manner, and may safely be bent.

A catheter of the present invention has a main lumen, and sub-lumens each of which having a diameter smaller than that of the main lumen. The plurality of sub-lumens are arranged along a circumference of the main lumen in a discrete manner. Each of the sub-lumens has an operating wire, which is fixed to the distal end section of the catheter, laid therethrough in a freely slidable manner.

According to a more specific embodiment of the present invention, the catheter may be configured so as to bend, when the proximal end(s) of the operating wire(s) is pulled to apply tensile force to the distal end section, the distal end section thereof towards the sub-lumen(s) having the pulled operating wire(s) laid therethrough; whereas so as to apply, when the proximal end of the operating wire(s) is pushed into the catheter, substantially no pressing force from the operating wire(s) to the distal end section of the catheter.

According to a still more specific embodiment of the present invention, three or more sub-lumens, each of which having the operating wire laid therethrough, may be arranged along the circumference of the main lumen in a discrete manner.
Alternatively, two sub-lumens, each of which having the operating wire laid therethrough, may be arranged along the circumference of the main lumen so as to oppose each other.
Alternatively, an operating unit, configured to pull the operating wire(s) to bend the distal end section of the catheter, may be provided to the proximal end section of the catheter.
Each of the operating wires may be fixed at a middle position of the distal end section of the catheter, and the distal end section may have a shaping portion provided ahead of the operating wire, more closer to the end.
The distal end section may have marker components composed of a radiation-impermeable material provided respectively at the end thereof and at the middle position.
The shaping portion may have a two-dimensional or three-dimensional flexion geometry.
The distal end section of the catheter may have a flexion geometry, one of the operating wires may be provided to a inner contour side of the flexion geometry, and other one of the operating wires may be provided to a outer contour side of the flexion geometry.
The catheter may have flexibility step-wisely increased in the direction from the proximal end towards the distal end thereof.

Note that each of various constituents of the present invention may not always necessarily be configured as an independent entity, and instead a plurality of constituents may configure a single component, a single constituents may be configured by a plurality of components, a certain constituent may be a part of other constituent, and a part of certain constituent may be shared with a part of other constituent.

According to the catheter of the present invention, by pulling the operating wire (s) respectively laid therethrough the sub-lumens, the distal end thereof may be bent towards the pulled operating wire(s). Since pushing of the operating wire is no longer necessary in the bending operation of the distal end section of the catheter, the bending operation may safely be proceeded. Since the operating wires are arranged at a plurality of positions around the main lumen in a discrete manner, the direction of bending of the distal end section may be controllable in a desirable manner.

### BRIEF DESCRIPTION OF THE DRAWINGS

The above and other objects, features and advantages of the present invention will be more apparent from the following description of certain preferred embodiments taken in conjunction with the accompanying drawings below.

FIG. 1 is a schematic vertical sectional view illustrating an exemplary catheter according to an embodiment of the present invention;
FIG. 2 is a sectional view taken along line II-II in FIG. 1;
FIG. 3 shows side elevations explaining operations of the catheter, wherein (a) is a schematic vertical sectional view illustrating the catheter in a natural status, and (b) and (c) are schematic vertical sectional views illustrating statuses of the catheter with its operating wire(s) pulled;
FIG. 4 (a) is schematic drawings explaining statuses of use of the catheter of this embodiment, (b) is schematic drawings explaining statuses of use of the catheter of this embodiment, and (c) is schematic drawings explaining statuses of use of the catheter of this embodiment;
FIG. 5 is a schematic vertical sectional view illustrating a catheter according to a second embodiment of the present invention;
FIG. 6 is a schematic transverse sectional view illustrating the distal end section of a catheter according to a third embodiment of the present invention;
FIG. 7(a) is a schematic vertical sectional view illustrating a catheter according to a fourth embodiment of the present invention, (b) is an enlarged view of the distal end section, and (c) is an enlarged view of the distal end section according to a modified example;
FIG. 8 is a schematic vertical sectional view illustrating a status of insertion, into a blood vessel, of the catheter of the fourth embodiment;
FIG. 9(a) is a schematic vertical sectional view illustrating a catheter according to a fifth embodiment of the present invention, (b) is a schematic drawing illustrating a status of bending of a shaping portion in this embodiment, and (c) is a schematic drawing illustrating a status of pulling of the operating wire in this embodiment;
FIG. 10 is a schematic drawing illustrating a status of insertion, into a branch of blood vessel, of the catheter of this embodiment; and
FIG. 11 (a) is a schematic drawing illustrating the distal end section of a catheter according to a sixth embodiment, and (b) is a schematic drawing illustrating the distal end section of a catheter according to a seventh embodiment.

### DESCRIPTION OF THE EMBODIMENTS

Embodiments of the present invention will be explained referring to the attached drawings. Not that all similar constituents in all drawings will be given similar reference numerals or symbols, so as to appropriately avoid repetitive explanation.

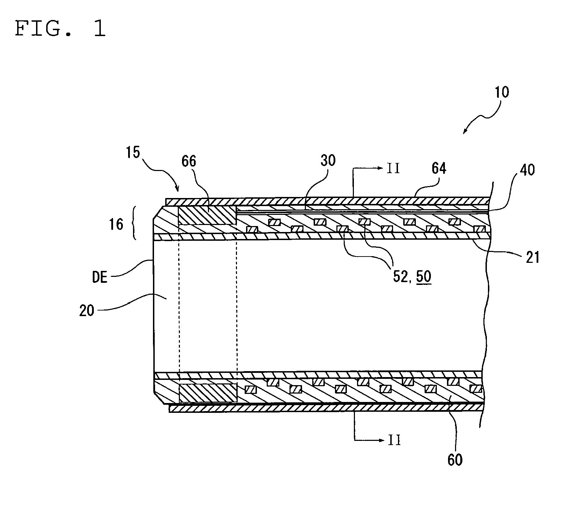

FIG. 1 is a schematic vertical sectional view illustrating a catheter 10 according to a first embodiment of the present invention, taken in the longitudinal direction. The distal end (tip) is directed to the left of drawing, and the proximal end (base) is directed to the right, although not illustrated in the drawing.

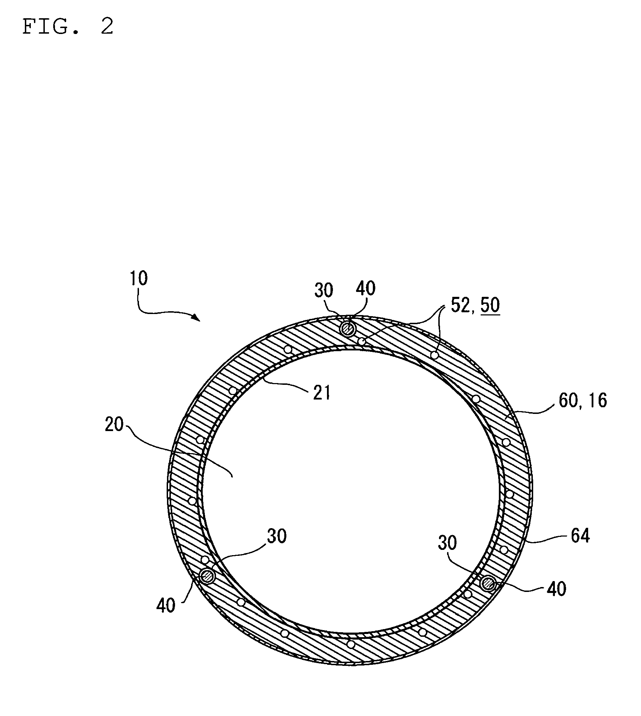

FIG. 2 is a sectional view (transverse cross section) taken along line II-II in FIG. 1.

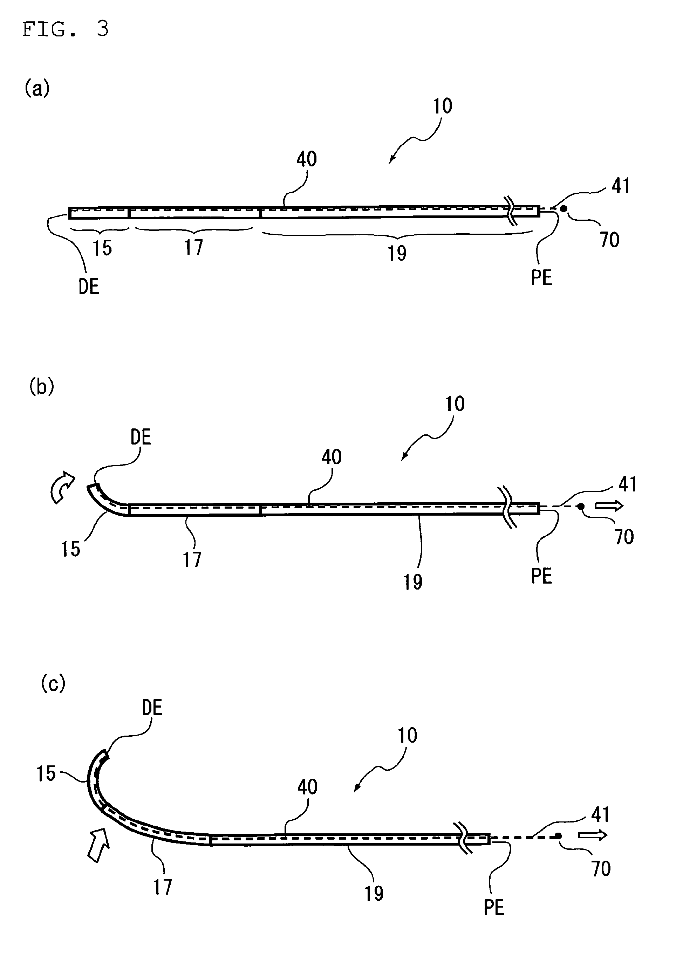

FIG. 3 shows side elevations explaining operations of the catheter 10 of this embodiment. FIG. 3(a) is a schematic vertical sectional view illustrating the catheter 10 in a natural status, FIG. 3 (b) is a schematic vertical sectional view illustrating the catheter 10 with its operating wire(s) 40 slightly pulled, and FIG. 3(c) is a schematic vertical sectional view illustrating the catheter 10 with its operating wire(s) 40 further pulled. In these drawings, only one operating wire out of three operating wires 40 in this embodiment is illustrated.

First, the catheter 10 of this embodiment will be outlined.
The catheter 10 of this embodiment has a main lumen 20, and sub-lumens 30 each of which having a diameter smaller than that of the main lumen 20.
The catheter 10 has a plurality of sub-lumens 30 arranged along a circumference of the main lumen 20 in a discrete manner, and each of the sub-lumens 30 has an operating wire 40, fixed to the distal end section 15 of the catheter 10, laid therethrough in a freely slidable manner.

Next, the catheter 10 of this embodiment will be detailed.
In the catheter 10 of this embodiment, tensile force is applied to the distal end section 15 thereof when proximal end(s) 41 of the operating wire(s) 40 is pulled, and thereby the distal end section 15 bends towards the sub-lumen(s) 30 having the pulled operating wire(s) laid therethrough.
On the other hand, when the proximal end(s) 41 of the operating wire (s) 40 is pushed into the catheter 10, substantially no pressing force is applied from the pushed operating wire(s) 40 to the distal end section 15 of the catheter 10.

Note that the distal end section 15 of the catheter 10 herein means a predetermined length of region of the catheter 10 which includes the distal end DE. Similarly, the proximal end section 19 of the catheter 10 herein means a predetermined length of region of the catheter 10 which includes the proximal end PE.
Bending of the catheter 10 herein means curving or flexion of the catheter 10 in a part thereof or over the entire portion thereof. The flexion and curving will not be discriminated in the present invention. In other words, the term "flexion" will be used in the present invention, irrespective of magnitude of radius of curvature.

The catheter 10 of this embodiment has three or more sub-lumens 30, each of which having the operating wire 40 laid therethrough, arranged along the circumference of the main lumen 20 in a discrete manner. FIG. 2 more specifically illustrates an embodiment having three sub-lumens 30 arranged at 120° intervals around the main lumen 20.

As illustrated in FIG. 1, the catheter 10 of this embodiment has a tubular inner layer 21 which is composed of a resin material and has the main lumen 20 formed therein; a braid layer 50 configured by a wire 52 woven around the inner layer 21; and an outer layer 60 which is composed of a resin material same as, or different from that composing the inner layer 21, formed around the inner layer 21 so as to enclose the braid layer 50.
In the catheter 10, the sub-lumens 30, each of which having the operating wire 40 laid therethrough, are formed inside the outer layer 60 and outside the braid layer 50.

It is now defined that the body of the catheter 10 composed of the resin material, having the main lumen 20 and the sub-lumens 30 formed therein, will be referred to as a sheath 16.
Each sub-lumen 30 is provided in the longitudinal direction of the catheter 10 (left/right direction in FIG. 1 and FIG. 3), and opens at least the proximal end section 19 of the catheter 10. Each proximal ends 41 of each operating wire 40 protrudes out from the proximal end section 19.

By providing the sub-lumens 30 having the operating wires 40 laid therethrough while being spaced from the main lumen 20, any drug and so on delivered therethrough or any optical component inserted therethrough will successfully be prevented from leaking into the sub-lumens 30.
By providing the sub-lumens 30 outside the braid layer 50 as seen in this embodiment, the inner portion of the braid layer 50, or the main lumen 20, may be protected from the sliding operating wires 40. Accordingly, even if the operating wires 40 should drop off from the distal end section 15 of the catheter 10, the operating wires 40 will not break the circumferential wall of the main lumen 20.

Around the outer layer 60, a hydrophilic coated layer 64 having a lubricated outer surface is optionally provided as the outermost layer of the catheter 10.
To the distal end section 15 of the catheter 10, a ring-form marker component 66 which is composed of a material impermeable against radioactive ray such as X-ray, is provided. More specifically, the marker component 66 may be composed of a metal material such as platinum. The marker component 66 in this embodiment is provided around the main lumen 20 and inside the outer layer 60.

The end (distal end) of each operating wire 40 is fixed to the distal end section 15 of the catheter 10. Mode of fixation of the end of each operating wire 40 to the distal end section 15 is not specifically limited. For example, the end of each operating wire 40 may be tied to the marker component 66, may be fused with the distal end section 15 of the sheath 16, or may be adhered and thereby fixed to the marker component 66 or to the distal end section 15 of the sheath 16 using an adhesive.

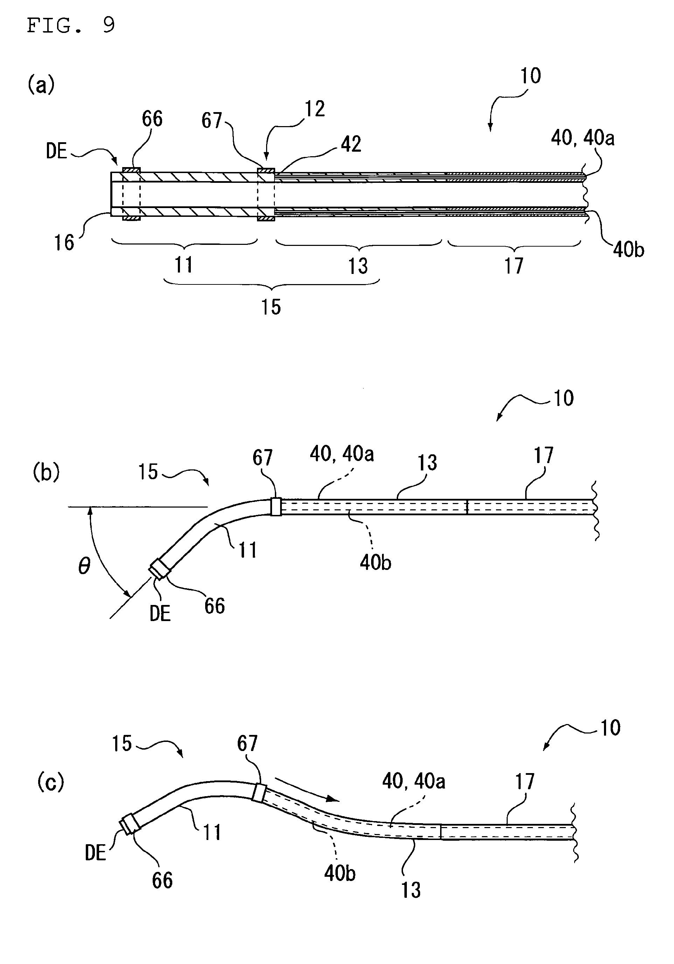

As described later, the catheter 10 may alternatively be provided with a plurality of marker components 66, 67. Still alternatively, the end of each operating wire 40 may be fixed to the middle position 12 of the distal end section 15 of the catheter 10, or may be fixed to the distal end DE (see FIG. 9).

The inner layer 21 may be configured using, for example, a fluorine-containing thermoplastic polymer material. More specifically, polytetrafluoroethylene (PTFE), polyvinylidene fluoride (PVDF), and perfluoroalkoxy resin (PFA) may be adoptable.
Adoption of the fluorine-containing resin to the inner layer 21 improves the delivery performance of the catheter 10, in the process of delivering such as a contrast medium or a medical solution through the main lumen 20 to a diseased site.

Thermoplastic polymers may widely be adoptable to the outer layer 60. For example, in addition to polyimide (PI), polyamide imide (PAI) and polyethylene terephthalate (PET), also polyethylene (PE), polyamide (PA), nylon elastomer, polyurethane (PU), ethylene-vinyl acetate resin (EVA), polyvinyl chloride (PVC) or polypropylene (PP) may be adoptable.

To the wire 52 composing the braid layer 50, metal thin wire composed of stainless steel (SUS) or nickel-titanium alloy, or polymer thin fiber composed of PI, PAI or PET may be adoptable.
Cross-sectional geometry of the wire 52 is not specifically limited, while allowing adoption of round wire and flat wire.

Various methods may be adoptable to insert the operating wires 40 through the sub-lumens 30. For example, the operating wires 40 may be inserted from one side of the sheath 16 of the catheter 10 having the sub-lumens 30 preliminarily formed therethrough. Alternatively, the operating wires 40 may be extruded together with a resin material in the process of extrusion molding of the sheath 16, so as to insert them into the sub-lumens 30.

In the process of extruding the operating wires 40 together with the resin material to insert them into the sub-lumens 30, the operating wires 40 are required to have a heat resistance represented by a temperature not lower than the melting temperature of the resin material composing the sheath 16. Specific examples of materials adoptable to this sort of operating wires 40 include polymer fibers composed of polyetheretherketone (PEEK), polyphenylene sulfide (PPS), polybutylene terephthalate (PBT), PI or PTFE; or metal wires composed of SUS, steel wire having corrosion-resistant coating, titanium or titanium alloy.
On the other hand, for the case where the operating wires 40 are not specifically required to have heat resistance, such as for the case where the operating wires 40 are inserted into the sub-lumens 30 of the preliminarily-formed sheath 16, materials adoptable herein include PVDF, high-density polyethylene (HDPE) and polyester, in addition to the above-described materials.

Hydrophilic materials such as polyvinyl alcohol (PVA) and polyvinyl pyrrolidone may be adoptable to the coated layer 64.

As illustrated in FIG. 3, the catheter 10 has an operating unit 70, which is configured to pull the operating wire(s) to bend the distal end section 15 of the catheter 10, provided to the proximal end section 19 of the catheter 10. The operating unit 70 independently pulls one of the plurality of operating wires 40, or concomitantly pulls two or more of them, so as to bend the distal end section 15.
In the catheter 10 of this embodiment, when the proximal end 41 of the operating wire 40 is pulled towards the base (rightward in FIG. 3), tensile force is applied through the marker component 66 to the distal end section 15 of the catheter 10, and thereby the distal end section 15 bends towards the sub-lumen 30 having the thus-pulled operating wire 40 laid therethrough.
When the catheter 10 is desired to be bent in an intermediate direction between the adjacent sub-lumens 30, it is good enough to pull two operating wires 40 laid through two sub-lumens 30 arranged on both sides of the desired direction.

According to the catheter 10 of this embodiment, the distal end section 15 may be bent in any directions with a 360 degree circle, by independently controlling the length of pulling of three operating wires 40. In this way, the direction of advancement of the catheter 10 may be controlled only by pulling of the operating wires 40 by the operating unit 70, rather than by torque control which applies torque over the entire body of the catheter 10 so as to bend the distal end section 15 to a predetermined direction.

As illustrated in FIG. 3, the catheter 10 of this embodiment has flexibility which stepwisely increases from the proximal end PE side towards the distal end DE side.
More specifically, as illustrated in FIG. 3(a), the catheter 10 is sectioned into the distal end section 15, an intermediate section 17 and the proximal end section 19 aligned in the longitudinal direction. Flexibility is larger in the intermediate section 17 than in the proximal end section 19, and is still larger in the distal end section 15 than in the intermediate section 17.
The flexibility of the catheter 10 herein means readiness of bending under unit load applied in the radial direction.

The flexibility of the catheter 10 may be varied in various ways. For example, thickness of the sheath 16 may be adjusted in a decreasing order from the proximal end section 19, through the intermediate section 17, to the distal end section 15. Alternatively, braid density of the braid layer 50 may be adjusted in a decreasing order from the proximal end section 19, through the intermediate section 17, to the distal end section 15.

By making the catheter 10 most flexible in the distal end section 15 as described in this embodiment, compliance of the distal end section 15 to pulling of the operating wire 40 may be improved as illustrated in FIG. 3(b), and thereby the distal end section 15 may readily be bent towards the pulled operating wire 40.
For the case where the amount of pulling of the operating wire 40 is increased as illustrated in FIG. 3(c), not only the distal end section 15 but also the intermediate section 17 may be bent.
On the other hand, by making the catheter 10 more rigid in the proximal end section 19 where a maximum load of bending moment will be applied due to the own weight of the catheter 10, the catheter 10 may be improved in the stiffness, and may keep the geometrical stability.

Alternatively, the flexibility of the catheter 10 continuously increases at least over a partial longitudinal range, in the direction from the proximal end PE towards the distal end DE.
More specifically, in the proximal end section 19, hardness is continuously increased towards the proximal end PE.
Accordingly, even if the catheter 10 is largely bent as illustrated in FIG. 3(c), the intermediate section 17 will not be prevented from deforming while being constrained by the rigidity of the proximal end section 19. In other words, according to this embodiment, the intermediate section 17 may thoroughly be bent and deformed, while keeping a sufficient level of moment resistance in the vicinity of the proximal end PE of the catheter 10.

Representative dimensions of the catheter 10 in this embodiment will be explained.
The main lumen 20 may be approximately 200 to 300 µm in radius, the inner layer 21 may be approximately 10 to 30 µm thick, the outer layer 60 may be approximately 100 to 150 µm thick, and the braid layer 50 may be approximately 20 to 30 µm thick. Radius measured between the axial center of the catheter 10 and the center of each sub-lumen 30 may be around 300 to 350 µm, inner diameter of each sub-lumen 30 may be 40 to 100 µm, and diameter of each operating wire 40 may be 30 to 60 µm. Outermost diameter of the catheter 10 may be around 350 to 450 µm.
In other words, the outer diameter of the catheter 10 of this embodiment is smaller than 1 mm, and may therefore be insertable in blood vessels such as celiac artery, and peripheral arteries such as hepatic artery branch and internal carotid artery branch. Since the catheter 10 of this embodiment is freely controllable in the direction of advancement by pulling the operating wire (s) 40, so that the catheter 10 may be allowed to proceed in a desired direction even in branched blood vessels.

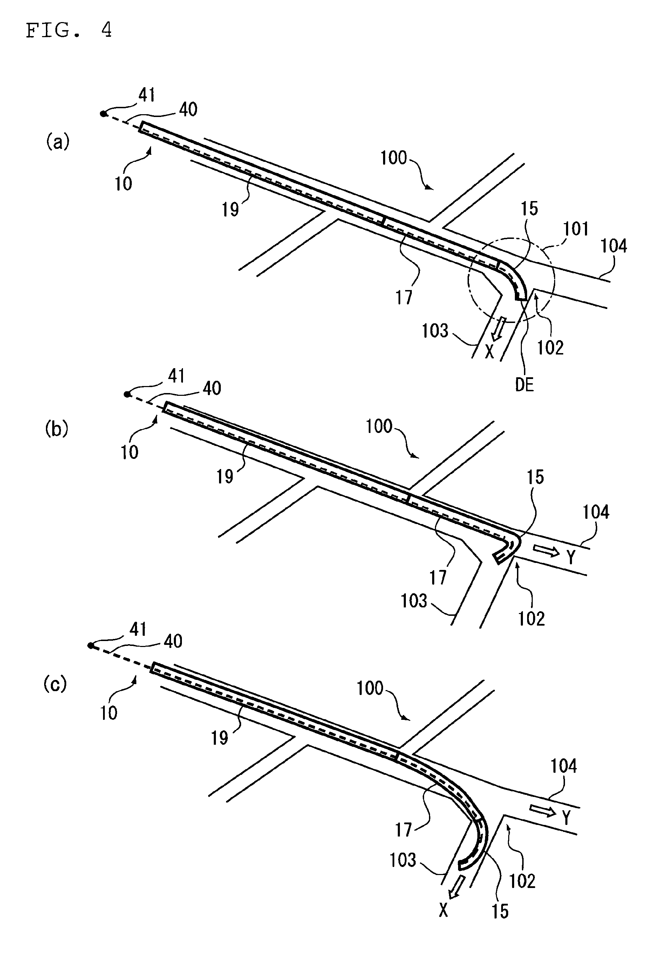

FIGs. 4(a) to (c) are schematic drawing explaining statuses of use of the catheter 10 of this embodiment.
FIG. 4 (a) illustrates a status of the catheter 10 inserted in a blood vessel 100, where the distal end DE came around a branched portion 101 of the blood vessel 100.
Now, a trail will be made to allow the catheter 10 to proceed from the branched portion 101 into a blood vessel branch 103, in direction X as indicated by the arrow in the drawing. Assume now that one or two operating wires 40, out of three, of the catheter 10 are pulled so as to bend the distal end section 15 towards direction X, as illustrated in FIG. 4(a).

However, since the distal end section 15 of the catheter 10 is highly flexible, so that the distal end section 15 may be folded at the corner portion 102 of the blood vessel 100 as illustrated in FIG. 4(b), and thereby the catheter 10 may undesirably proceed in direction Y which lies in the direction of extension of the principal blood vessel 104.
Now, according to the catheter 10 of this embodiment, not only the distal end section 15 but also the intermediate section 17 may be bent towards direction X by further pulling the proximal end(s) 41 of the operating wire(s) 40 as illustrated in FIG. 4(c).

In this way, an overall direction of advancement of the catheter 10 may be altered from direction Y representing the direction of extension of the principal blood vessel 104, into direction X representing the direction of extension of the blood vessel branch 103.
By allowing the distal end DE of the catheter 10 to advance into the blood vessel branch 103 to a sufficient depth, the intermediate section 17 and the proximal end section 19 follow thereafter into the blood vessel branch 103. Since the intermediate section 17 and the proximal end section 19 have bending rigidity larger than that of the distal end section 15, so that the catheter 10 is no longer anticipated to be bent and advance into the principal blood vessel 104, even if the intermediate section 17 and the proximal end section 19 should be brought into contact with the corner portion 102.
As is clear from the above, the catheter 10 of this embodiment may be allowed to advance in a desired direction, even in branched blood vessel or peripheral arteries.

In the catheter 10 of this embodiment, angle of bending of the distal end section 15 preferably exceeds 90°. If so, the catheter 10 may be allowed to proceed even into artery branch which is branched out at an acute angle such as forming a U-turn in the blood vessel 100.

Note that the present invention is not restricted to the above-described embodiment, while allowing various modifications and improvements so far as the objects of the present invention may be accomplished.

For example, while the above-described embodiment dealt with the case where the sub-lumens 30 are formed outside the braid layer 50, the present invention is not restricted thereto.

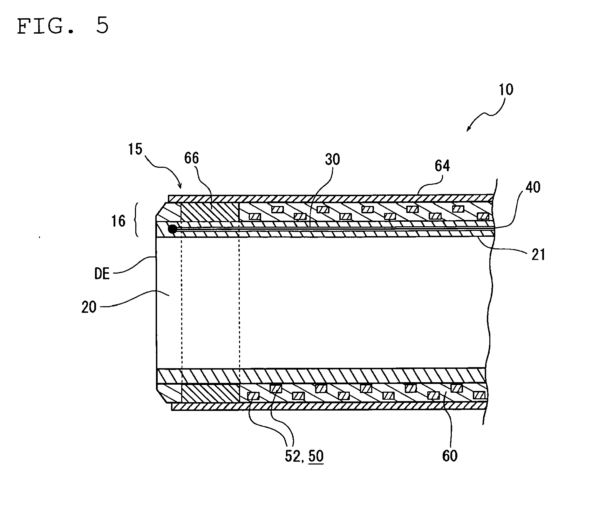

FIG. 5 is a schematic vertical sectional view of the catheter 10 according to a second embodiment.

The catheter 10 of this embodiment has a tubular inner layer 21 which is composed of a resin material and has the main lumen 20 formed therein; a braid layer 50 configured by a wire 52 woven around the inner layer 21; and an outer layer 60 which is composed of a resin material same as, or different from that composing the inner layer 21, formed around the inner layer 21 so as to enclose the braid layer 50.
The catheter 10 has the sub-lumens 30, each of which having the operating wire 40 laid therethrough, formed inside the inner layer 21.

In this embodiment, the end of each operating wire 40 laid therethrough each sub-lumen 30 is fused with the distal end section 15 of the inner layer 21.

In the catheter 10 of this embodiment, since the outer circumferences of the sub-lumens 30 are protected by the braid layer 50, so that the operating wires 40 are prevented from breaking through the outer layer 60 to be exposed to the outside, even if applied with excessive tensile force during operation and accidentally detached from the distal end section 15.

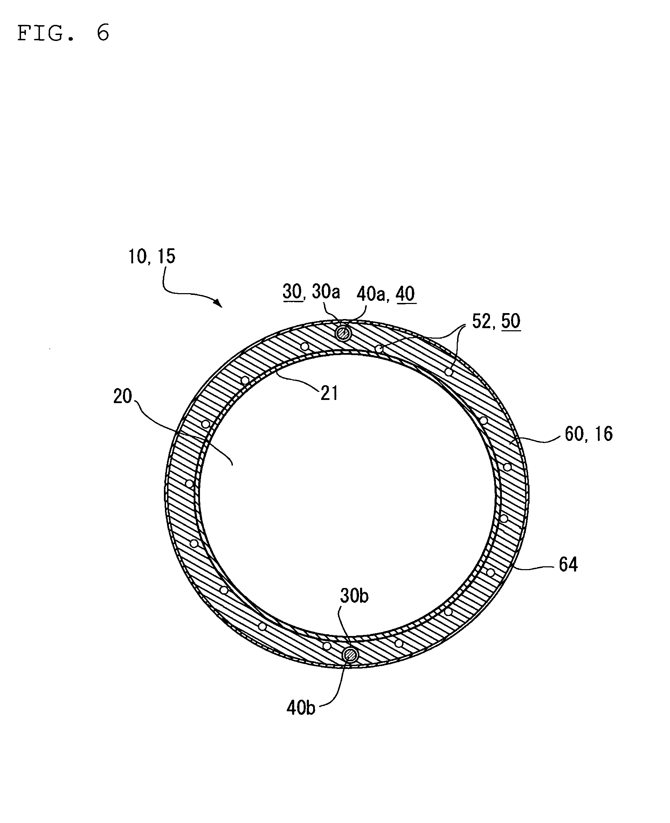

FIG. 6 is a schematic transverse sectional view illustrating the distal end 15 of a catheter according to a third embodiment of the present invention;
In the catheter 10 of this embodiment, two sub-lumens 30 (30a, 30b), each of which having the operating wires 40 (40a, 40b) laid therethrough, are arranged along the circumference of the main lumen 20 so as to oppose each other.

More specifically, in this embodiment, the sub-lumen 30a and the sub-lumen 30b are formed so as to oppose each other at 180° intervals while placing the axial center of the catheter 10 in between. The sub-lumen 30a has the operating wire 40a laid therethrough, and the sub-lumen 30b has the operating wire 40b laid therethrough.

According to the catheter 10 of this embodiment, when the operating unit 70 (see FIG. 3) is operated to pull the operating wire 40a towards the proximal end PE side (depth-wise direction behind the sheet of FIG. 6), the distal end section 15 of the catheter 10 bends upward in FIG. 6. On the other hand, when the operating unit 70 is operated to pull the operating wire 40b towards the proximal end PE side, the distal end section 15 of the catheter 10 bends downward in FIG. 6.

Accordingly, by applying torque for rotating the catheter 10 up to 90° while keeping the operating wire 40a or the operating wire 40b pulled, the operator may bend the distal end section 15 of the catheter 10 in a desired direction.
For example, if the distal end section 15 of the catheter 10 is desired to bend rightward in FIG. 6, it is good enough to apply torque so as to rotate the entire body of the catheter 10 by 90° clockwise, while pulling the operating wire 40a so as to keep the distal end section 15 bent upward in the drawing. Alternatively, the torque may be applied so as to rotate the entire body of the catheter 10 by 90° counterclockwise, while pulling the operating wire 40b so as to keep the distal end section 15 bent downward in the drawing.

In short, according to the catheter 10 of this embodiment, the distal end section 15 may be bent in a desired direction, while suppressing the rotation, under applied torque, of the entire body of the catheter 10 to as much as 90°. Accordingly, the operator may quickly direct the distal end section 15 of the catheter 10 towards a desired direction.

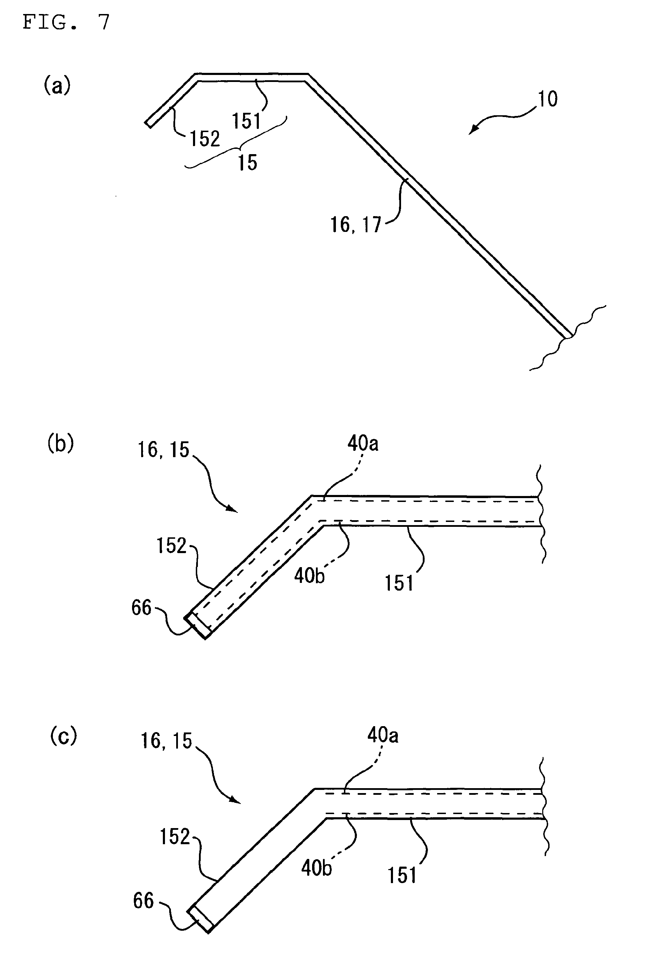

FIG. 7 (a) is a schematic vertical sectional view of the catheter 10 according to a fourth embodiment of the present invention, and FIG. 4(b) is an enlarged view of the distal end section 15. FIG. 4(c) is an enlarged view of the distal end section 15 according to a modified example of this embodiment.

The catheter 10 of this embodiment is different from that of the third embodiment, in that the distal end section 15 has a flexion geometry. The catheter 10 of this embodiment has one operating wire 40a provided along a outer contour of flexion, and the other operating wire 40b provided along a inner contour of flexion.

More specifically, the catheter 10 of this embodiment has the shaped distal end section 15 of the sheath 16, wherein a first flexion 151 and a second flexion 152 are formed in succession as continued from the intermediate section 17 on the base side. The first flexion 151 and the second flexion 152 reside in the same plane. In other words, the distal end section 15 in this embodiment has a two-dimensional, multi-step flexion geometry. The first flexion 151 and the second flexion 152 respectively have approximately 45° flexion.

The first flexion 151 is longer than the second flexion 152. Assuming now that the sheath 16 has an outer diameter of 700 to 900 µm, the first flexion 151 may typically be 10 to 15 mm long, and the second flexion 152 may be 3 to 10 mm long.

As illustrated in FIG. 7(b), the operating wire 40a and the operating wire 40b are laid through the sheath 16 up to the vicinity of the marker component 66 which is provided to the endmost of the distal end section 15, and are respectively tied to the sheath 16 at the end thereof. In this configuration, when the operating wire 40a is pulled rightward in the drawing, the second flexion 152 is lifted upward in the drawing, and thereby the angle of flexion between the first flexion 151 and the second flexion 152 decreases. If the operating wire 40a is further pulled, the second flexion 152 and the first flexion 151 are lifted upward in the drawing, and thereby the angle of flexion between the first flexion 151 and the intermediate section 17 (see FIG. 7(a)) decreases.

Alternatively, in this embodiment, the operating wire 40a and the operating wire 40b may be tied with the sheath 16 at around the end portion of the first flexion 151 as illustrated in FIG. 7(c). In this configuration, if the operating wire 40a is pulled, the first flexion 151 is lifted upward in the drawing while keeping the angle of flexion between the second flexion 152 and the first flexion 151 unchanged, and thereby the angle of flexion away from the intermediate section 17 (see FIG. 7(a)) decreases.

On the other hand, by pulling the operating wire 40b, the first flexion 151 and the second flexion 152 may be flexed in the direction opposite to that described in the above, and thereby the angle of flexion of the distal end section 15 may increase.

Note that the sheath 16 does not immediately recover the natural status, after pulling either the operating wire 40a or the operating wire 40b by a predetermined force, and then releasing the force to zero. In other words, there is some time lag before the pulled sheath 16 recovers the natural status. According to this embodiment, the sheath 16 deformed by pulling one operating wire may quickly recover the natural status, by releasing the force of pulling, and then pulling the other operating wire.

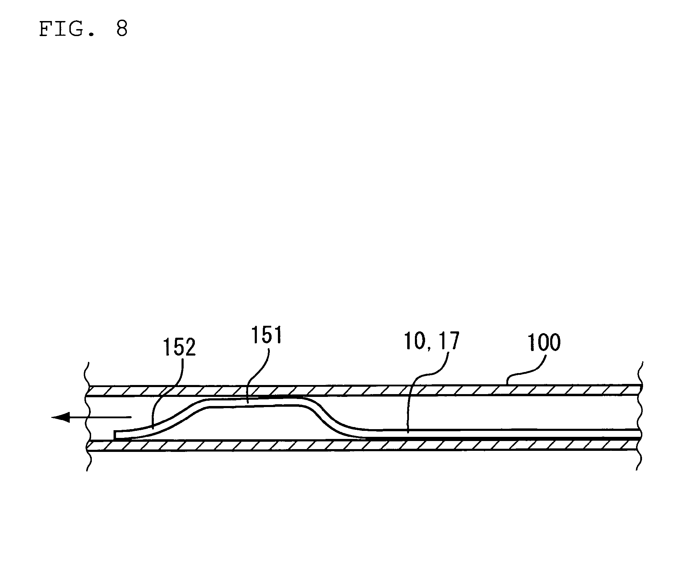

FIG. 8 is a schematic vertical sectional view illustrating a status of catheter 10 of this embodiment in the process of insertion into the blood vessel 100. By pulling the operating wire 40a (not shown in the drawings) so as to moderate the angle of flexion of the second flexion 152, the catheter 10 of this embodiment, shaped in the end section thereof to have the flexion geometry, may be inserted into the relatively-narrow blood vessel 100.

When the second flexion 152 came around the blood vessel branch 103 (see the individual drawings in FIG. 4) of the blood vessel 100, the second flexion 152 may readily be inserted into the blood vessel branch 103, by rotating the catheter 10 while applying torque so as to adjust the direction of flexion of the second flexion 152 to the direction of branching of the blood vessel branch 103, and then moderating the force of pulling of the operating wire 40a. In this operation, by pulling the operating wire 40b, the second flexion 152 may more readily be inserted into the blood vessel branch 103.

While the distal end section 15 having the two-step flexion geometry was exemplified in this embodiment, the present invention is not limited thereto. The distal end section 15 may arbitrarily be shaped to have a U-form, L-form, S-form, Ω-form, spiral form, or combination of these forms, without special limitations on the angle of flexion and the radius of curvature.

The catheter 10 of this embodiment may conveniently be formed by preliminarily inserting the operating wire 40a and the operating wire 40b into the straight tubular sheath 16, tying the end portions thereof to the sheath 16, and by deforming the distal end section 15 of the sheath 16 to give the flexion geometry.
The operating wires 40a, 40b of this embodiment have a softening temperature (melting temperature) higher than the softening temperature of the sheath 16. Accordingly, in the process of shaping of the distal end section 15 of the sheath 16, the end portion of the sheath 16 may preferably be heated to a temperature not lower than the softening temperature of the sheath 16 but lower than the softening temperature (melting temperature) of the operating wires 40a, 40b, so as to flex the sheath 16 to a desired direction, and may be cooled thereafter.

A point of time the distal end section 15 needs be subjected to the shaping is not specifically limited. More specifically, the catheter 10 may preliminarily be shaped (pre-shaped) before being shipped as the final product, or may be shaped (re-shaped) on site immediately before the catheter 10 is inserted into body cavity such as blood vessel.

FIG. 9 (a) is a schematic vertical sectional view of the catheter 10 according to the fifth embodiment of the present invention.
The catheter 10 of this embodiment has the operating wires 40 (40a, 40b) fixed to the middle position 12 of the distal end section 15 of the catheter 10. The distal end section 15 of the catheter 15 has a shaping portion 11 provided ahead of the operating wires 40 (more closer to the distal end DE).

The catheter 10 illustrated in the drawing has the shaping portion 11 given in a straight form, for the re-shaping for the future.

The distal end section 15 of the catheter 10 has the marker components 66, 67 composed of a radiation-impermeable material respectively provided at the end thereof (distal end DE) and at the middle position 12.

The middle position 12 where the marker component 67 is provided may not necessarily be the absolute center of the distal end section 15.

The catheter 10 of this embodiment has flexibility which stepwisely increases from the proximal end (right side of the drawing) towards the distal end DE side. More specifically, the distal end section 15 has a bending elastic modulus of the sheath 16 smaller than that of the intermediate section 17, and flexibility of the sheath 16 higher than that of the intermediate section 17.

In the catheter 10 of this embodiment, the distal end section 15 is further divided into the shaping portion 11 and an active portion 13. The active portion 13 is a region of the distal end section 15, which is provided on one side of the fixed ends 42 of the operating wires 40 more closer to the base. On the other hand, the shaping portion 11 is a region of the distal end section 15, which is provided on the other side of the fixed ends 42 more closer to the distal end DE.
The marker component 67 is provided in the vicinity of the fixed ends 42 of the operating wires 40. The fixed ends 42 of the operating wires 40 may be tied with the marker component 67, or may slightly be spaced therefrom. In other words, the marker component 67 is provided at a boundary region between the shaping portion 11 and the active portion 13.

FIG. 9(b) is a schematic drawing illustrating a status of the catheter 10 of this embodiment, having the shaping portion 11 flexed by pre-shaping or re-shaping. The shaping portion 11 in this embodiment flexes downward in the drawing, from the marker component 67 towards the distal end DE.

The angle of flexion θ of the shaping portion 11 is preferably set to 40 to 50°. By the setting, the catheter 10 may appropriately be inserted into blood vessels which branch or flex at various angles.
In the catheter 10 of this embodiment, the axial length may preferably be set to 3 to 10 mm for the shaping portion 11; 10 to 20 mm for the active portion 13; and 20 mm or longer for the intermediate section 17.

In the distal end section 15 of the catheter 10, the shaping portion 11 has a flexion geometry, wherein one operating wire 40b is provided to the inner contour side of flexion, and the other operating wire 40a is provided to the outer contour side of flexion.

FIG. 9 (c) is a schematic drawing illustrating a status observed when the operating wire 40a provided to the outer contour side of the shaping portion 11 is pulled. When the operating wire 40a is pulled towards the base side as indicated by the arrow, the active portion 13 bends towards the operating wire 40a. Since the shaping portion 11, which is provided ahead of the marker components 67, has no operating wire 40 laid therethrough, so that the shaping portion 11 may keep the flexion geometry. In other words, the catheter 10 of this embodiment may flex the active portion 13 while keeping the flexion geometry of the shaping portion 11.

Since the intermediate section 17 in this embodiment has a bending rigidity sufficiently larger than that of the active portion 13, so that substantially only the active portion 13 bends when the operating wires 40 are pulled. The shaping portion 11 and the active portion 13 have the bending rigidity and radial dimension same with those of the sheath 16.

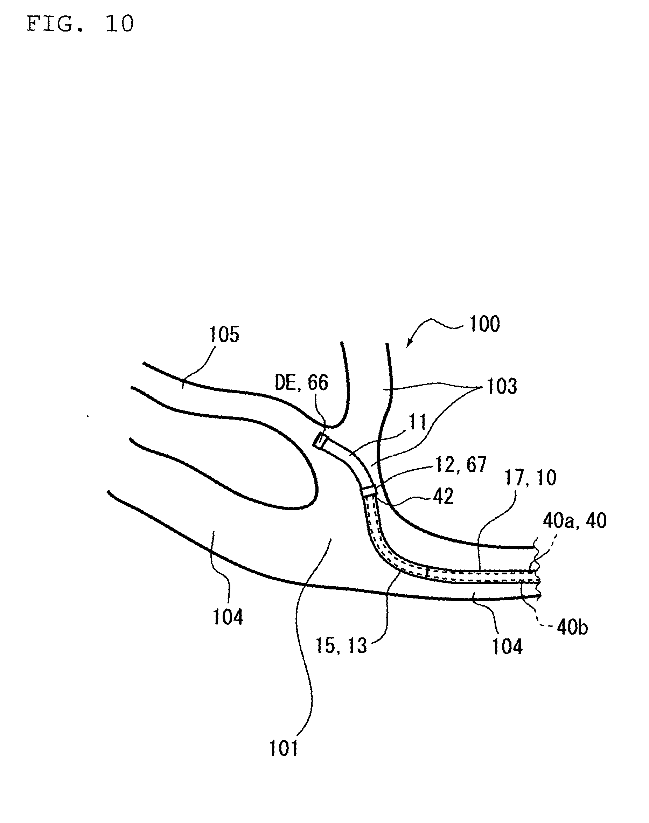

FIG. 10 is a schematic drawing illustrating a status of insertion of the catheter 10 of this embodiment, illustrated in FIG. 9(b), into a blood vessel branch 105. The drawing illustrates the status in which the shaping portion 11 of the catheter 10, which was inserted from the right of the drawing into the principal blood vessel 104 of the blood vessel 100, came around the branched portion 101 of the blood vessel 100.

The catheter 10 of this embodiment, inserted into the body cavity such as blood vessel 100, may visually be observed under extracorporeal irradiation of radioactive ray such as X-ray, since it is recognizable by the distal end DE and the middle position 12 attached with the marker component 66 and the marker component 67, respectively. Accordingly, approach of the shaping portion 11 to the branched portion 101 of the blood vessel 100 may be detected.

At the branched portion 101, the first blood vessel branch 103 branches out from the principal blood vessel 104. The branched portion 101 also has the second blood vessel branch 105 which branches out closely next to the branching site of the first blood vessel branch 103.
For this reason, in order to allow the catheter 10 to advance from the principal blood vessel 104 thorough the first blood vessel branch 103 into the second blood vessel branch 105, the catheter 10 is necessarily flexed nearly in a S-form.

Since the catheter 10 of this embodiment has the fixed ends 42 of the operating wires 40 on one side of the shaping portion 11 more closer to the base, and the shaping portion 11 is therefore not loaded with the force of pulling of the operating wires 40, so that the active portion 13 may be bent while keeping the flexion geometry of the shaping portion 11. Accordingly, as illustrated in FIG. 10, the catheter 10 may be deformed nearly into the S-form by pulling the operating wire 40a provided to the outer contour side of the flexion of the shaping portion 11, and thereby the distal end DE having the marker component 66 provided thereto may appropriately be inserted into the second blood vessel branch 105.

Note that, by virtue of the flexion geometry of the shaping portion 11, the catheter 10 may be inserted into the simply branched blood vessel 100, only by adjusting the direction of flexion of the shaping portion 11 to the direction of branching by rotating the catheter 10 under applied torque, without pulling the operating wires 40. This enables quick insertion into the blood vessel branch 103.

The catheter 10 of this embodiment has the marker components 66, 67 respectively at both ends of the shaping portion 11. Each of the marker components 66, 67 has an annular geometry. Position and orientation of the marker components 66, 67 may therefore be confirmed under irradiation of radioactive ray. Accordingly, not only the position of the distal end DE having the marker component 66 attached thereto, but also the middle position 12 having the marker component 67 attached thereto, and still also the orientation of the shaping portion 11 may be detectable.
In this way, the catheter 10 may selectively be inserted into the blood vessel branches 103, 105 which branch out at various angles.

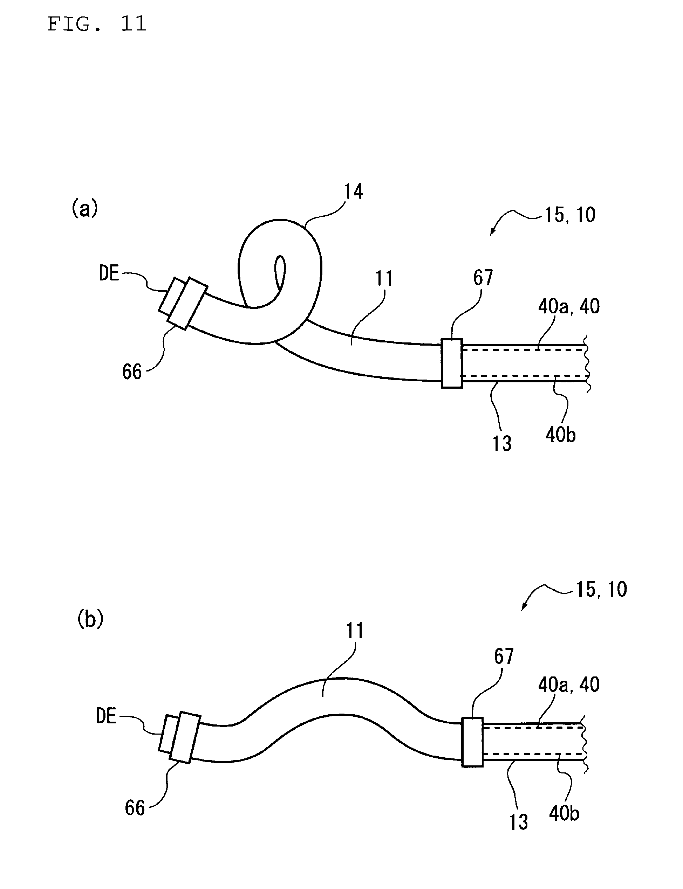

FIG. 11 (a) is a schematic drawing illustrating the distal end section 15 of the catheter 10 according to a sixth embodiment of the present invention. FIG. 11 (b) is a schematic drawing of the distal end section 15 of the catheter 10 according to a seventh embodiment of the present invention.

In each of these catheters 10, the shaping portion 11 has a two-dimensional, or three-dimensional flexion geometry. The catheter 10 of the sixth embodiment has the shaping portion 11 spirally formed to give a loop 14, and appears as a three-dimensional flexion geometry. The catheter 10 of the seventh embodiment has the shaping portion 11 formed to give a wavy form, and appears as a two-dimensional flexion geometry.

Similarly to the fifth embodiment, also the catheters 10 of the sixth and seventh embodiments have the operating wires 40 fixed to the marker components 67, laid through the active portion 13 towards the base side. Accordingly, the shaping portion 11 is prevented from being applied with bending load if the operating wire (s) 40 is pulled.

The catheter 10 of the sixth embodiment is particularly convenient for insertion into a looped blood vessel configured by tumor cells or the like. More specifically, when the distal end DE of the catheter 10 illustrated in FIG. 11(a) is inserted into the looped blood vessel, the loop 14 may be inserted conforming to the loop geometry of the blood vessel.
It is to be noted that the shaping portion 11 is not applied with force of pulling, even if the operating wire(s) 40 is pulled so as to direct the end (marker component 67) of the active portion 13 towards a predetermined blood vessel branch, so that the shaping portion 11 may freely bend and deform by virtue of its flexibility. Accordingly, the loop 14 of the shaping portion 11 may flexibly conform to the looped blood vessel, and thereby the catheter 10 may be inserted into the blood vessel without applying any excessive load thereto.

The same will apply also to the catheter 10 of the seventh embodiment illustrated in FIG. 11(b). The catheter 10 may appropriately be inserted into the wavy blood vessel, irrespective of whether the operating wire(s) 40 is pulled to bend the active portion 13 or not.

This application claims priority right based on Japanese Patent Application No. 2008-332204 filed on December 26, 2008, and Japanese Patent Application No. 2009-108473 filed on April 27, 2009, the entire contents of which are incorporated hereinto by reference.

## Claims

1. A catheter having a main lumen, and sub-lumens each of which having a diameter smaller than that of the main lumen,
the plurality of sub-lumens being arranged along a circumference of the main lumen in a discrete manner, and
each of the sub-lumens having an operating wire, which is fixed to the distal end section of the catheter, laid therethrough in a freely slidable manner.

2. The catheter according to Claim 1, configured so as to bend, when the proximal end(s) of the operating wire (s) is pulled to apply tensile force to the distal end section, the distal end section towards the sub-lumen(s) having the pulled operating wire(s) laid therethrough
whereas so as to apply, when the proximal end (s) of the operating wire (s) is pushed into the catheter, substantially no pressing force from the pushed operating wire(s) to the distal end section of the catheter.

3. The catheter according to Claim 1 or 2,
configured to have three or more sub-lumens, each of which having the operating wire laid therethrough, arranged along the circumference of the main lumen in a discrete manner.

4. The catheter according to Claim 1 or 2,
configured to have two sub-lumens, each of which having the operating wire laid therethrough, arranged along the circumference of the main lumen so as to oppose each other.

5. The catheter according to any one of Claims 1 to 4, having an operating unit, configured to pull the operating wire (s) to bend the distal end section of the catheter, provided to the proximal end section of the catheter.

6. The catheter according to any one of Claims 1 to 5,
wherein each of the operating wires is fixed at a middle position of the distal end section of the catheter, and
the distal end section has a shaping portion provided ahead of the operating wire, more closer to the end.

7. The catheter according to Claim 6,
wherein the distal end section has marker components composed of a radiation-impermeable material provided respectively at the end thereof and at the middle position.

8. The catheter according to Claim 6 or 7,
wherein the shaping portion has a two-dimensional or three-dimensional flexion geometry.

9. The catheter according to any one of Claims 1 to 8,
wherein the distal end section of the catheter has a flexion geometry, and
one of the operating wires is provided to a inner contour side of the flexion geometry, and other one of the operating wires is provided to a outer contour side of the flexion geometry.

10. The catheter according to any one of Claims 1 to 9, having flexibility step-wisely varied in the direction from the proximal end towards the distal end of the catheter.

11. The catheter according to any one of Claims 1 to 10, having flexibility continuously increased at least over a partial longitudinal range, in the direction from the proximal end towards the distal end of the catheter.

12. The catheter according to any one of Claims 1 to 11, comprising a tubular inner layer which is composed of a resin material and has the main lumen formed therein; a braid layer configured by a wire woven around the inner layer; and an outer layer which is composed of a resin material same as, or different from that composing the inner layer, formed around the inner layer so as to enclose the braid layer,
wherein the sub-lumens, each of which having the operating wire laid therethrough, are formed in the inner layer.

13. The catheter according to any one of Claims 1 to 11, comprising a tubular inner layer which is composed of a resin material and has the main lumen formed therein; a braid layer configured by a wire woven around the inner layer; and an outer layer which is composed of a resin material same as, or different from that composing the inner layer, formed around the inner layer so as to enclose the braid layer,
wherein the sub-lumens, each of which having the operating wire laid therethrough, are formed inside the outer layer and outside the braid layer.
